(19) Europäisches Patentamt
European Patent Office
Office européen des brevets

(11) Publication number: 0 222 561 B1

(12) EUROPEAN PATENT SPECIFICATION

(45) Date of publication of patent specification: 08.05.91 Bulletin 91/19

(51) Int. Cl.$^5$: C12P 13/02, C12P 41/00

(21) Application number: 86308486.9

(22) Date of filing: 30.10.86

# (54) Process for enzymatic separation of optical isomers of 2-aminobutanol.

(30) Priority: 31.10.85 IT 2269585

(43) Date of publication of application: 20.05.87 Bulletin 87/21

(45) Publication of the grant of the patent: 08.05.91 Bulletin 91/19

(84) Designated Contracting States: AT BE CH DE ES FR GB LI NL SE

(56) References cited:
PATENT ABSTRACTS OF JAPAN, vol. 8, no. 126 (C-228)[1563], 13th June 1984; & JP-A-59 39 294 (HAMARI YAKUHIN KOGYO K.K.) 03-03-1984
PATENT ABSTRACTS OF JAPAN, vol. 8, no. 34 (C-210)[1471], 15th February 1984; & JP-A-58 198 296 (CHISSO K.K.) 18-11-1983
THE JOURNAL OF ORGANIC CHEMISTRY, vol. 52, no. 23, 13th November 1987, pages 5079-5082, American Chemical Society; F. FRANCALANCI et al.: "Lipase-Catalyzed resolution of Chiral 2-amino 1-alcohols"

(73) Proprietor: Montedison S.p.A.
31, Foro Buonaparte
I-20121 Milan (IT)

(72) Inventor: Francalanci, Franco
7, via Galileo Ferraris
I-28100 Novara (IT)
Inventor: Cesti, Pietro
51, via Torino
I-28069 S. Martino di Trecate Novara (IT)
Inventor: Foá, Marco
19, via Del Sabbione
I-28100 Novara (IT)
Inventor: Martinengo, Tiziano
Via Alessandro Mossotti
I-28064 Carpignano Sesia Novara (IT)

(74) Representative: Whalley, Kevin et al
MARKS & CLERK 57/60 Lincoln's Inn Fields
London WC2A 3LS (GB)



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention relates to a process for the enzymatic separation of optical isomers ($S^+$) and ($R^-$) of racemic 2-aminobutanol ($S^+$, $R^-$).

More particularly the invention relates to a biotechnological process to obtain 2-aminobutanol, substantially in the form of the optical isomer ($S^+$). 2-aminobutanol in its ($S^+$) form is a useful intermediate for the synthesis of an important antitubercular agent (+)-2,2'-(ethylenediimino)-bis-[1-butanol] (ETHAMBUTOL).

A few methods are known for the separation of the optical antipodes of racemic 2-aminobutanol ($S^+$, $R^-$) through formation of diastereoisomeric salts by means of optically active acids (US-A-3,944,608 ; EP-A-36,265; Hung. Teljes 19,369). The known methods for splitting the two optical isomers are however complicated, they need expensive optically active reactants, require a careful control of the reaction conditions, and involve several crystallization steps of the products, and therefore they are disadvantageous from an industrial point of view.

It is therefore desirable to provide a method allowing the splitting of the optical isomers of 2-aminobutanol in a simple, efficient and economic way.

The present invention aims to provide a method for the separation of the optical isomers of 2-aminobutanol, and in particular to obtain isomer ($S^+$) in a simple, economic way, which method is generally free from the drawbacks of the known methods of separation.

It has now been found that this aim may be attained by a biotechnological process of enzymatic unsymmetrical esterification of a suitable ($S^-$, $R^+$) N-carbamoyl derivative of racemic 2-aminobutanol.

In practice use is made of an enzyme belonging to the lipase class, capable of giving rise to the esterification reaction in a stereoselective way on the form ($R^+$) of N-carbamoyl derivative of racemic 2-aminobutanol. The non-reacted N-carbamoyl derivative in the form ($S^-$) may be separated easily from the obtained ester and successively hydrolyzed to 2-aminobutanol ($S^+$).

The present invention provides a process for the enzymatic separation of optical isomers ($S^+$) and ($R^-$) of racemic 2-aminobutanol ($S^+$, $R^-$) by reacting the ($S^-$, $R^+$) N-carbamoyl derivative of 2-aminobutanol of formula:

($S^-$, $R^+$) 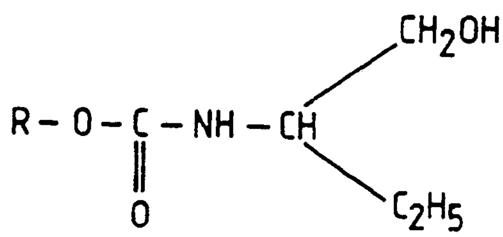 (I)

wherein R is a $C_1$-$C_3$ alkyl group or a benzyl group, with an ester of formula :

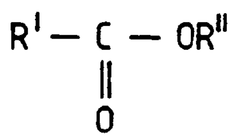 (II)

wherein R', R'', which may be the same or different, are $C_1$-$C_6$ alkyl groups, at a temperature from 0° to 60°C, in the presence of an enzyme, stable in the reaction medium, selected from the lipase class, capable of giving rise, in a selective way, to the esterification reaction on form ($R^+$) of N-carbamoyl derivative (I), allowing ($S^-$)N-carbamoyl derivative to remain substantially unchanged, and separating, according to known processes, non-reacted ($S^-$)N-carbamoyl derivative from the obtained ester, and hydrolyzing ($S^-$)N-carbamoyl derivative to give rise to ($S^+$)2-aminobutanol, preferably hydrolyzing separately ($S^-$)N-carbamoyl derivative and the obtained ester, to give rise to ($S^+$)2-aminobutanol and ($R^-$)2-aminobutanol respectively.

In practice it has been found that, only by protecting the amine group of 2-aminobutanol and, in general, of 2-amino-1-alkanols, through formation of a carbamoyl NH-CO-group, the esterification reaction can take place in a stereoselective way in the presence of an enzyme, since otherwise the reaction is not stereoselective and leads mainly to the formation of the corresponding amide.

N-carbamoyl derivatives (I) may be synthesized, according to known methods, under Schotten-Baumann conditions (for instance Jerry March, Advanced Org. Chem. : Reactions, Mechanics and Structure, Mc Graw-Hill Ed.), in practice by reacting racemic 2-aminobutanol with an alkylchloroformate in an aqueous alkaline

medium, according to reaction :

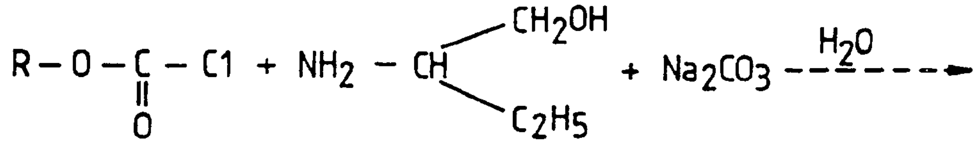


(III) (IV)

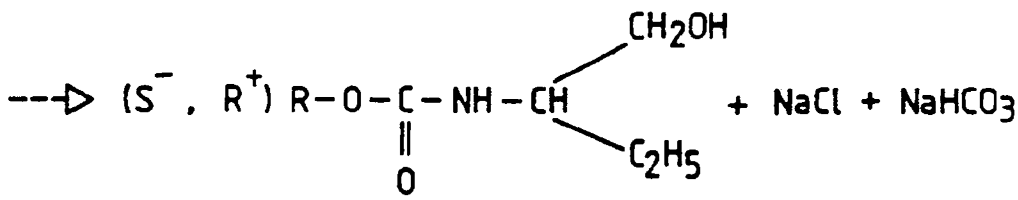

(I)

wherein R is a $C_1$-$C_3$ alkyl group or benzyl group, preferably a $-C_2H_5$ group.

($S^-$, $R^+$)N-carbamoyl derivative (I) is reacted, according to the process of the present invention, with an ester of a carboxylic acid (II), in the presence of the enzyme, according to reaction :

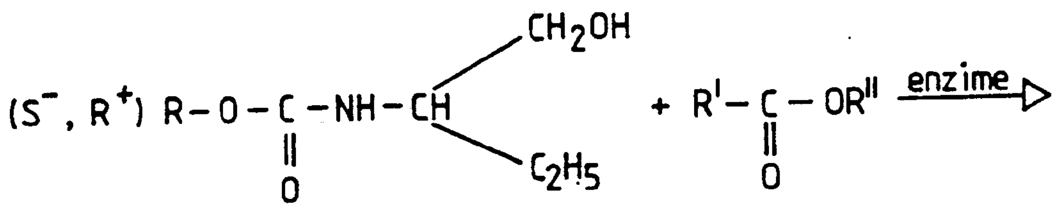


(I) (II)

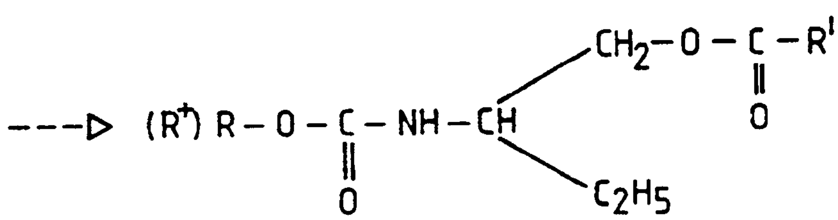

(V)

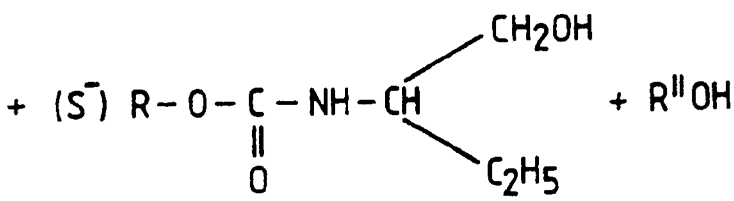

(VI)

wherein R has the same meaning as specified hereinbefore and R', R", which may be the same or different, are $C_1$-$C_6$ alkyl groups, preferably R' = $-CH_3$ and R" = $-C_2H_5$.

The ester of carboxylic acid (II) is used in excess, as it is used in the reaction both as reactant and as solvent, and in particular use is made of molar ratios of ester (II) : N-carbamoyl derivative (I) of from 1 : 5 to 1 : 100, more preferably from 1 : 10 to 1 : 30.

The enzyme that may be used in the process according to the invention is a lipase, in particular pancreatin or steapsin, stable in the reaction medium, capable of giving rise to the esterification reaction in a stereoselective way between ($R^+$)N-carbamoyl derivative (I) and ester (II). Such lipases are commercially available, optionally immobilized on an inert substrate of different nature.

In the reaction use is preferably made of ratios by weight of enzyme : N-carbamoyl derivative (I) of from 1 : 1 to 1 : 20.

The process of stereoselective esterification is carried out by strongly stirring the reaction mixture of reactants (I) and (II), the ester (II) in excess acting as solvent and enzyme, at a temperature from 0° to 60°C, preferably from 20° to 30°C.

When the reaction of stereoselective esterification is over, there is commenced the filtration of the solid phase consisting essentially of the enzyme, that afterwards may be purified by means of one or more washings and recovered substantially without any activity loss.

From the filtrate consisting of the organic phase of the reaction, ($S^-$)N-carbamoyl derivative (VI) is subsequently separated from esterified ($R^+$)N-carbamoyl derivative (V), by using conventional methods, for instance by column chromatography, by fractional distillation or, more advantageously, by exploiting their different solubility in water, by means of washing(s) with water of the organic phase and subsequent separation of the aqueous phase, containing dissolved ($S^-$)N-carbamoyl derivative (VI), from the organic phase consisting essentially of esterified ($R^+$)N-carbamoyl derivative (V) and of the non-reacted ester of carboxylic acid (II).

($S^-$)N-carbamoyl derivative (VI) is subsequently hydrolyzed according to a known process, for instance by alkaline hydrolysis to give rise to ($S^+$)2-aminobutanol.

If desired, esterified ($R^+$)N-carbamoyl derivative (V) may be subjected to hydrolysis to give rise to ($R^-$)2-aminobutanol.

The invention will be further described with reference to the following illustrative Examples.

<u>Example 1</u>

A mixture of 8.9 g of ($S^+$, $R^-$) 2-aminobutanol and 10.8 g of $Na_2CO_3$ in 40 ml of $H_2O$ was treated at 0°C with 10.8 g of ethylchloroformate. The whole was kept under stirring at room temperature for 2 hours, and then the product was extracted by means of chloroform and the solution was dried on calcium chloride.

After solvent evaporation, there were obtained 14.8 g of ($S^-$, $R^+$) 2-amino-N-ethoxycarbonylbutanol (yield 92%) that may be later purified by distillation at reduced pressure (b.p. 105-107°C at 0.5 mm Hg) [NMR $\delta$ = 5.2 d 1 H ; 4.1 q 2H ; 3.4-3.7 m 4H ; 1.35-1.65 m 2H ; 1.25 t 3H ; 0.95 t 3H – in $COCl_3$].

<u>Example 2</u>

0.9 g of lipase coming from a pork pancreas (PPL catalogue Sigma No L 3126 35-70 U/mg protein) were added to a solution of 3 g of ($S^-$, $R^+$)2-amino-N-ethoxycarbonylbutanol in 30 ml of ethylacetate. The whole was kept under stirring for 110 hours at room temperature. The enzyme was then removed by filtration, the organic solution was extracted 5 times by means of water, and from said solution, after solvent evaporation, 1.2 g of ($R^+$) 2-amino-N-ethoxycarbonylbutanol acetate were recovered ; by extracting again the washing waters by means of toluene a further 0.5 g of the same product were obtained, which therefore was recovered with a yield of 44% and had an $[\alpha]_D$ = +18.5° (c = 2 in ethanol) corresponding to e.e. = 74.4%.

By further extraction of the waters by means of chloroform there were recovered 1.3 g of ($S^-$) 2-amino-N-ethoxycarbonylbutanol (yield 43%) having $[\alpha]_D$ = –21° (c = 2 in ethanol) corresponding to e.e. = 99%.

<u>Example 3</u>

1.3 g of ($S^-$)2-amino-N-ethoxycarbonylbutanol having $[\alpha]_D$ = –21.0° (c = 2 in ethanol) were treated for 2 hours at 60°C with 5 ml of sodium carbonate at 30%. The aqueous solution was distilled, thereby recovering 0.6 g of ($S^+$)2-aminobutanol having $[\alpha]_D$ = +12.3° (c = 2 in ethanol) corresponding to e.e. = 98.4%.

Example 4

1 g of pancreatin (UNIBIOS 57 U FIP/mg) was added to a solution of 3 g of ($S^-$, $R^+$)2-amino-N-ethoxycarbonylbutanol in 30 ml of ethylacetate. The whole was kept under stirring for 40 hours at room temperature and the raw product was treated as described in Example 2, thereby obtaining 1.9 g of ($R^+$)2-amino-N-ethoxycarbonylbutanol acetate (yield 49.1%) having $[\alpha]_D$ = +15.6° (c = 2 in ethanol) corresponding to e.e. = 62.6% and 1.25 g of ($S^-$) 2-amino-N-ethoxycarbonylbutanol (yield 41.7%) having $[\alpha]_D$ = 20.6° corresponding to e.e. = 97.6%.

By hydrolysis of the latter product as described in Example 3, there were obtained 0.5 g of ($S^+$) 2-aminobutanol having $(a)_D$ = +12.2° (c = 2 in ethanol) corresponding to e.e. = 97.6%.

Example 5

1 g of lipase PPL of Example 2 was added to a solution of 3 g of ($S^-$, $R^+$) 2-amino-N-benzyloxycarbonylbutanol in 30 ml of ethylacetate. The whole was kept under stirring at room temperature for 48 hours. The enzyme was then removed by filtration and the products were separated by chromatography on a silica gel column (eluent hexane : ethyl acetate = 5 : 1). There were obtained 1.3 g of ($R^+$) 2-amino-N-benzyloxycarbonylbutanol acetate (yield 36.4%) having $[\alpha]_D$ = +13° (c = 2 in ethanol) and 1.7 g of ($S^-$) 2-amino-N-benzyloxycarbonylbutanol (yield 56.5%) having $[\alpha]_D$ = –13.8° (c = 2 in ethanol).

Example 6

3 g of ($R^+$) 2-amino-N-benzyloxycarbonylbutanol acetate were dissolved in 45 ml of ethanol and treated at room temperature with 100 ml of Pd/C at 10% under hydrogen atmosphere. After 6 hours under stirring, the mixture was filtered, the solvent was evaporated, and the residue was treated with 5 ml of $H_2SO_4$ at 10% and kept at 80°C for 2 hours. The pH was brought to 10 by means of NaOH and the aqueous solution was distilled, thereby obtaining 0.5 g of ($R^-$) 2-aminobutanol having $[\alpha]_D$ = –11.3° (c = 2 in ethanol), corresponding to e.e. = 90.4%.

Example 7

2 g of ($S^+$, $R^-$) 2-aminobutanol were dissolved in 30 ml of ethyl acetate and treated with 1 g of lipase PPL of Example 2, at room temperature for 60 hours. The reaction mixture was filtered, evaporated until a dry residue was obtained and the products were separated by chromatography on a silica gel column (eluent ether : methanol = 1 : 1). 1.1 g of ($S^+$, $R^-$) 2-aminobutanol (yield 55%) and 0.9 g of (RS) racemic 2-aminobutanol (yield 30.5%) were recovered.

## Claims

1. A process for the enzymatic separation of optical isomers ($S^+$) and ($R^-$) of racemic 2-aminobutanol ($S^+$, $R^-$), characterized by reacting the ($S^-$, $R^+$) N-carbamoyl derivative of 2-amınobutanol of formula :

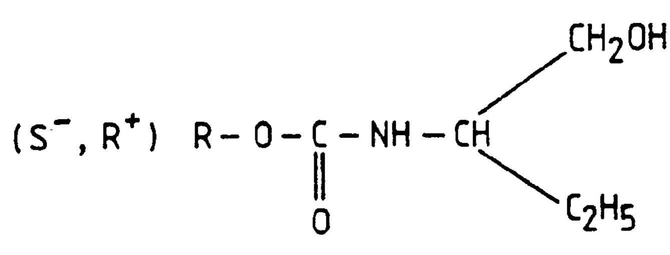

(I)

wherein R is a $C_1$-$C_3$ alkyl group or a benzyl group, with an ester of formula :

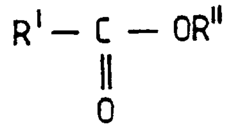

(II)

wherein R′, R″, which may be the same or different, are alkyl groups having from 1 to 6 carbon atoms, at a temperature from 0° to 60°C, in the presence of an enzyme, stable in the reaction medium, selected from the lipase class, capable of giving rise, in a selective way, to an esterification reaction on form ($R^+$) of N-carbamoyl derivative (I), allowing ($S^-$) N-carbamoyl derivative to be substantially unchanged, separating non-reacted ($S^-$) N-carbamoyl derivative from the obtained ester, and hydrolyzing separately ($S^-$) N-carbamoyl derivative and the obtained ester to give rise to ($S^+$) 2-aminobutanol and ($R^-$)2-aminobutanol respectively.

2. A process as claimed in claim 1, characterized in that R is $-C_2H_5$, R′ is $-CH_3$ and R″ is $-C_2H_5$.

3. A process as claimed in claim 1 or 2, characterized in that the molar ratio of ester (II) : N-carbamoyl derivative (I) is from 1 : 5 to 1 : 100.

4. A process as claimed in claim 3, characterized in that the molar ratio of ester (II) : N-carbamoyl derivative (I) is from 1 : 10 to 1 : 30.

5. A process as claimed in any of claims 1 to 4, characterized in that the lipase is selected from pancreatin and steapsin.

6. A process as claimed in any of claims 1 to 5, characterized in that the molar ratio by weight of lipase : N-carbamoyl derivative (I) is from 1 : 1 to 1 : 20.

7. A process as claimed in any of claims 1 to 6, characterized in that the reaction temperature is from 20° to 30°C.

8. A process as claimed in any of claims 1 to 7, characterized in that non-reacted ($S^-$) N-carbamoyl derivative is separated from the reaction mixture by means of washing(s) with water.

## Ansprüche

1. Verfahren zur enzymatischen Trennung der optisch aktiven Isomere ($S^+$) und ($R^-$) von racemischem 2-Aminobutanol ($S^+$, $R^-$), dadurch gekennzeichnet, daß man das ($S^-$, $R^+$)-N-Carbamoyl-Derivat von 2-Aminobutanol der Formel

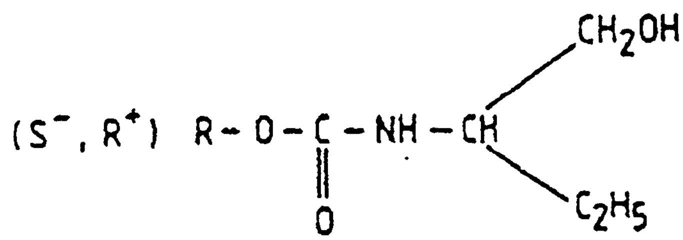

(I)

in der R einen $C_1$-$C_3$-Alkyl- oder Benzylrest bedeutet, mit einem Ester der Formel

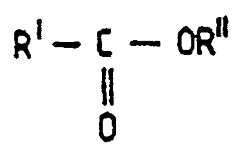

(II)

in der R′ und R″, die gleich oder verschieden sein können, Alkylreste mit 1 bis 6 Kohlenstoffatomen sind, bei einer Temperatur zwischen 0 und 60°C in Gegenwart eines Enzyms umsetzt,
wobei das Enzym, das im Reaktionsmedium stabil ist und aus der Klasse der Lipasen ausgewählt wird, geeignet ist, in selektiver Weise die Veresterung der ($R^+$)-Form des N-Carbamoyl-Derivats (I) zu bewirken, während das ($S^-$)-N-Carbamoyl-Derivat im wesentlichen unverändert bleibt,
das unumgesetzte ($S^-$)-N-Carbamoyl-Derivat vom erhaltenen Ester abtrennt und das ($S^-$)-N-CarbamoylDerivat sowie den erhaltenen Ester getrennt hydrolysiert, um ($S^+$)-2-Aminobutanol bzw. ($R^-$)-2-Aminobutanol zu erhalten.

2. Verfahren nach Anspruch 1, dadurch gekennzeichnet, daß R $-C_2H_5$, R′ $-CH_3$ und R″ $-C_2H_5$ bedeutet.

3. Verfahren nach Anspruch 1 oder 2, dadurch gekennzeichnet, daß das Molverhältnis von Ester (II) zu N-Carbamoyl-Derivat (I) zwischen 1 : 5 und 1 : 100 liegt.

4. Verfahren nach Anspruch 3, dadurch gekennzeichnet, daß das Molverhältnis von Ester (II) zu N-Carbamoyl-Derivat (I) zwischen 1 : 10 und 1 : 30 liegt.

5. Verfahren gemäß irgendeinem der Ansprüche 1 bis 4, dadurch gekennzeichnet, daß die Lipase aus Pancreatin und Steapsin ausgewählt wird.

6. Verfahren gemäß irgendeinem der Ansprüche 1 bis 5, dadurch gekennzeichnet, daß das Gewichtsverhältnis von Lipase zu N-Carbamoyl-Derivat (I) zwischen 1 : 1 und 1 : 20 liegt.

7. Verfahren nach irgendeinem der Ansprüche 1 bis 6, dadurch gekennzeichnet, daß die Reaktionstemperatur zwischen 20 und 30°C liegt.

8. Verfahren gemäß irgendeinem der Ansprüche 1 bis 7, dadurch gekennzeichnet, daß das unumgesetzte ($S^-$)-N-Carbamoyl-Derivat von der Reaktionsmischung durch Waschen mit Wasser abgetrennt wird.

**Revendications**

1. Un procédé pour la séparation enzymatique d'isomères optiques ($S^+$) et ($R^-$) de 2-aminobutanol ($S^+$, $R^-$) racémique, caractérisé en ce que l'on fait réagir le dérivé ($S^-$, $R^+$) N-carbamoyl-2-aminobutanol de formule :

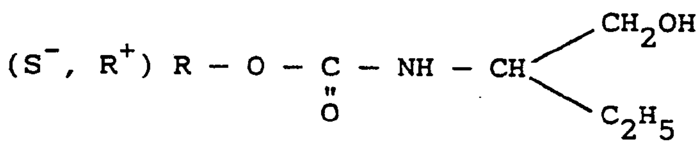 (I)

dans laquelle :

R est un radical alkyle comprenant 1 à 3 atomes de carbone ou un radical benzyle ;

avec un ester de formule :

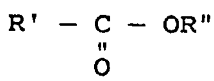 (II)

dans laquelle :

R′, R″ qui peuvent être identiques ou différents, sont des groupes alkyles comprenant de 1 à 6 atomes de carbone, à une température comprise entre 0 et 60°C, en présence d'un enzyme, stable dans le milieu réactionnel, choisi dans la classe des lipases, susceptibles de donner lieu, de façon sélective, à une réaction d'estérification sur la forme ($R^+$) du dérivé N-carbamoyle (I), en laissant le dérivé ($S^-$) N-carbamoyle pratiquement non modifié, en ce que l'on sépare le dérivé ($S^-$) N-carbamoyle n'ayant pas réagi de l'ester obtenu et en ce que l'on hydrolyse séparément le dérivé ($S^-$) N-carbamoyle et l'ester obtenu pour produire le ($S^+$) 2-aminobutanol et le ($R^-$) 2-aminobutanol respectivement.

2. Un procédé ainsi que revendiqué dans la revendication 1, caractérisé en ce que R est $-C_2H_5$, R′ est $-CH_3$ et R″ est $-C_2H_5$.

3. Un procédé ainsi que revendiqué dans la revendication 1 ou 2, caractérisé en ce que le rapport molaire ester (II)/dérivé N-carbamoyle (I) est compris entre 1/5 et 1/100.

4. Un procédé ainsi que revendiqué dans la revendication 1, caractérisé en ce que le rapport molaire ester (II)/dérivé N-carbamoyle (I) est compris entre 1/10 et 1/30.

5. Un procédé ainsi que revendiqué dans l'une quelconque des revendications 1 à 4, caractérisé en ce que le lipase consiste en pancréatine ou stéapsine.

6. Un procédé ainsi que revendiqué dans l'une quelconque des revendications 1 à 5, caractérisé en ce que le rapport molaire pondéral lipase/dérivé N-carbamoyle (I) est compris entre 1/1 et 1/20.

7. Un procédé ainsi que revendiqué dans l'une quelconque des revendications 1 à 6, caractérisé en ce que la température réactionnelle est comprise entre 20 et 30°C.

8. Un procédé ainsi que revendiqué dans l'une quelconque des revendications 1 à 7, caractérisé en ce que le dérivé ($S^-$) N-carbamoyle n'ayant pas réagi est séparé du milieu réactionnel par un ou plusieurs lavages à l'aide d'eau.